# FASCICULE DE BREVET EUROPEEN

(11) **EP 3 067 075 B1**
(45) Date de publication et mention de la délivrance du brevet: **16.10.2019**
(21) Numéro de dépôt: 16159051.8
(22) Date de dépôt: 07.03.2016
(51) Int. Cl.: A61L 27/28, A61F 2/07, A61F 2/82

(54) **ENDOPROTHÈSE TISSULAIRE ET PROCÉDÉ POUR SA RÉALISATION**
GEWEBE-ENDOPROTHESE UND VERFAHREN ZU IHRER ANWENDUNG
TISSUE STENT AND METHOD FOR MANUFACTURING SAME

(30) Priorité: 10.03.2015 FR 1500457
(43) Date de publication de la demande: 14.09.2016
(73) Titulaire: CARMAT, 78941 Vélizy Villacoublay cedex (FR)
(72) Inventeur: CAPEL, Antoine, 91380 CHILLY MAZARIN (FR); DUCROS, Clément, 78220 VIROFLAY (FR); POULETTY, Philippe, 75005 PARIS (FR); CADUDAL, Jean-Claude, 92140 CLAMART (FR)
(74) Mandataire: Gevers & Orès

(56) Documents cités:
- EP-A2- 1 586 286
- WO-A1-2010/024882
- WO-A2-02/49535
- WO-A2-03/007781
- US-A1- 2005 143 801
- US-A1- 2006 025 848
- US-A1- 2007 083 258

## Description

La présente invention concerne une endoprothèse tissulaire et un procédé de réalisation de cette endoprothèse.

Les endoprothèses tissulaires concernées par la présente invention peuvent, par exemple, être des endoprothèses vasculaires ou des prothèses de valves cardiaques, notamment des valves aortiques, mitrales ou tricuspides.

Par le document WO 03/007781 (PCT/US02/20037), on connaît déjà une endoprothèse vasculaire tissulaire comportant une structure de soutien expansible creuse, à surface ajourée, et une structure tissulaire interne de tissu biologique recouvrant la surface interne de ladite structure de soutien.

Ladite structure de soutien expansible creuse, généralement appelée stent, présente une mémoire de forme pour pouvoir être implantée dans un vaisseau dans un état replié et reprendre sa forme radialement expansée une fois en place dans ledit vaisseau, et sa surface est percée de jours, dus au fait que cette surface est généralement constituée par un grillage à mailles d'une matière telle que le Nitinol, le titane, etc... Quant à elle, la structure tissulaire interne est par exemple constituée par une membrane de péritoine, de plèvre ou de péricarde animal, qui est roulée pour pouvoir s'adapter à la forme de la surface interne de la structure de soutien, lorsque celle-ci présente sa forme radialement expansée. Il en résulte que cette structure interne de tissu biologique comporte obligatoirement au moins une ligne de jonction le long de laquelle se raccordent deux bords opposés de ladite membrane.

Dans cette endoprothèse vasculaire connue, les deux bords opposés ainsi raccordés sont solidarisés l'un de l'autre par des points de suture qui, d'une part, risquent d'engendrer des turbulences dans l'écoulement du sang dans l'endoprothèse et donc la formation de caillots et, d'autre part, ne peuvent assurer une étanchéité rigoureuse le long de ladite ligne de jonction. Pour tenter de remédier à ces inconvénients, il est proposé, dans ce document antérieur, de recouvrir la ligne des points de suture par une couche de colle biologique. Or, une telle colle biologique est, pour l'endoprothèse, un produit étranger procurant une adhésion purement chimique qui dénature le tissu biologique et qui est susceptible de s'émietter. Il en résulte alors que des particules de colle biologique, détachées de la couche de colle biologique recouvrant la ligne des points de suture, peuvent former des caillots sanguins conduisant à une embolie du patient.

Par ailleurs, dans cette endoprothèse connue, la structure tissulaire interne est de préférence fixée à ladite structure de soutien également à l'aide de points de suture. Il va de soi que ces points de suture supplémentaires peuvent également être à l'origine de caillots sanguins, comme expliqué ci-dessus pour les points de suture de la structure interne. Aussi, accessoirement, est-il proposé dans ce document de coller ladite structure tissulaire interne sur la structure de soutien à l'aide d'une colle biologique. Mais alors, on retrouve les risques d'embolie mentionnés ci-dessus dus à l'utilisation d'une telle colle biologique.

On remarquera que la réalisation des points de suture est de plus délicate et longue, de sorte que la production de ces endoprothèses est peu performante, avec de nombreux rebuts.

En outre, une fois placée en position expansée dans un vaisseau sanguin, la structure de soutien d'une endoprothèse est au contact dudit vaisseau, en pressant celui-ci radialement vers l'extérieur. Il en résulte des frottements risquant d'éroder la surface dudit vaisseau et pouvant conduire au déplacement, dans ledit vaisseau, de l'endoprothèse vasculaire à l'état expansé.

Une endoprothèse telle que celle du préambule de la revendication 1 est connue du document EP-A-1 586 286.

Par ailleurs, par les documents US 5 411 552 et EP 0 850 607 A1, on connaît une prothèse de valve cardiaque comportant une structure de soutien externe expansible à surface ajourée et une structure tissulaire interne couvrant au moins en partie la surface interne de ladite structure de soutien. Dans ces prothèses de valves connues, de façon semblable à ce qui a été décrit ci-dessus, la structure tissulaire interne est fixée à la structure de soutien externe par collage ou par réalisation de points de suture. Elles présentent donc tous les inconvénients mentionnés ci-dessus.

La présente invention a pour objet de remédier à tous ces inconvénients en décrivant une endoprothèse tissulaire parfaitement étanche, renforcée, ne risquant pas d'éroder les cellules des tissus d'un patient après implantation et ne comportant ni points de suture, ni colle biologique.

À cette fin, selon l'invention, l'endoprothèse tissulaire comportant :
- une structure de soutien expansible creuse à surface ajourée (stent) ; et
- une structure tissulaire interne couvrant au moins en partie la surface interne de ladite structure de soutien,
est remarquable en ce qu'elle comporte un revêtement externe constitué par une matière synthétique souple hémocompatible et ancré mécaniquement à ladite structure tissulaire interne, ladite structure de soutien étant emprisonnée au moins partiellement entre ledit revêtement externe et ladite structure tissulaire interne.

De préférence, un tel ancrage mécanique est dû au fait que la matière synthétique souple hémocompatible dudit revêtement externe imprègne au moins partiellement ladite structure tissulaire interne.

Ainsi, dans l'endoprothèse tissulaire conforme à la présente invention, ledit revêtement externe, qui est en une manière synthétique souple, pure ou chargée (inclusion, substance active,...), avantageusement un élastomère, de préférence un élastomère de polyuréthane ou de silicone, exerce plusieurs fonctions, sans pour autant, grâce à sa souplesse, gêner l'expansion de ladite structure de soutien expansible (stent) de sa position d'implantation repliée à sa position implantée expansée. En effet, ledit revêtement externe :
- qui a accès à ladite structure tissulaire interne à travers les jours de la surface de la structure de soutien, réalise avec cette structure interne un collage par ancrage mécanique dû à l'imprégnation de la structure interne par la matière synthétique souple dudit revêtement externe, ce collage, qui emprisonne ladite structure de soutien et la solidarise du revêtement externe et de la structure interne, permettant d'obtenir une très bonne adhésion mécanique, au contraire d'une colle biologique qui procure une adhésion purement chimique ;
- recouvre la ligne de jonction, résultat de la conformation du revêtement interne à partir d'une membrane, en adhérant aux bords en regard de cette ligne de jonction et assure de ce fait l'étanchéité de cette dernière ; et
- forme, pour l'endoprothèse conforme à la présente invention, un renfort externe souple protégeant, après implantation, les parois naturelles du patient contre l'usure résultant de frottements.

Avantageusement, l'épaisseur dudit revêtement externe est au moins de 0,1 mm et peut atteindre 5 mm. Éventuellement, cette épaisseur peut ne pas être uniforme et varier d'un endroit à l'autre dudit revêtement externe.

La structure tissulaire interne peut être constituée par un tissu biologique d'origine animale, par exemple du péricarde, ou bien par une matière syntétique, par exemple du polytétrafluoroéthylène
Conformément à la présente invention, pour réaliser ladite endoprothèse tissulaire, on met avantageusement en oeuvre un procédé remarquable en ce que :
- ladite structure tissulaire interne est introduite dans ladite structure de soutien de façon à couvrir de façon continue, uniforme, sans pli, ni surépaisseur, au moins une partie de la surface interne de ladite structure de soutien ;
- à l'aide d'une dispersion d'une matière synthétique souple hémocompatible dans un solvant, on recouvre la surface de ladite structure de soutien et les parties de la surface externe de ladite structure tissulaire interne accessibles à travers les jours de la surface de ladite structure de soutien, pour former un revêtement externe de ladite matière synthétique souple hémocompatible ancré mécaniquement dans ladite structure tissulaire interne ; et
- on extrait ledit solvant dudit revêtement externe.

Dans le cas où ladite structure tissulaire interne est formée par un tissu biologique fixé chimiquement cette structure tissulaire interne est soumise à une déshydratation partielle après fixation chimique et avant introduction dans ladite structure de soutien, puis est réhydratée pendant ou après l'extraction du solvant dudit revêtement externe.

Avantageusement, la concentration en poids de ladite matière synthétique dans la dispersion est comprise entre 10 et 30 %, de préférence entre 20 et 22 %. La viscosité de ladite dispersion est avantageusement comprise entre 500 et 1000 cP.

Les figures du dessin annexé feront bien comprendre comment l'invention peut être réalisée. Sur ces figures, des références identiques désignent des éléments semblables.
La figure 1 illustre schématiquement et partiellement une portion d'endoprothèse, pendant la mise en place de la structure tissulaire interne dans la structure de soutien expansible.
La figure 2 est une coupe longitudinale partielle, selon la ligne II-II de la figure 1, de la paroi de l'ensemble structure de soutien - structure tissulaire interne après assemblage de ces éléments.
La figure 3 correspond à la figure 2 et montre le revêtement externe réalisé sur ledit ensemble structure de soutien - structure tissulaire interne, conformément à la présente invention.

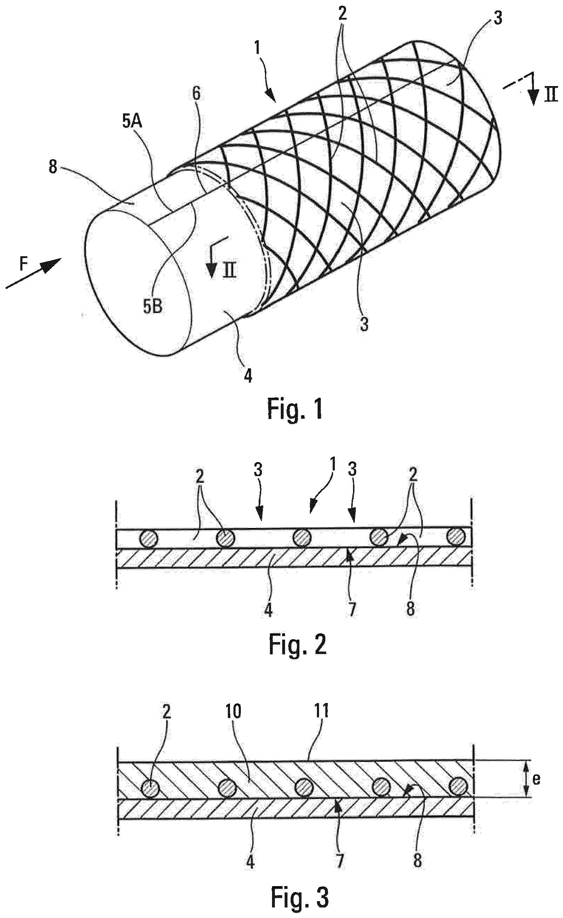

Sur la figure 1, on a représenté, en position déployée, une portion d'endoprothèse tissulaire, par exemple une endoprothèse vasculaire ou une endoprothèse de valve cardiaque, comportant :
- une structure de soutien 1 (stent) constituée par un grillage de fils 2 d'un alliage à mémoire de forme (acier, Nitinol, métal, polymère...), dont les mailles 3 forment des jours dans la surface de ladite structure de soutien, cette structure pouvant être symétrique ou asymétrique dans l'axe longitudinal ou latéral ; et
- une structure tissulaire interne 4, par exemple taillée dans une membrane (non représentée) de tissu biologique et roulée de façon que ses bords opposés 5A et 5B se raccordent le long d'une ligne de jonction 6, ce tissu biologique pouvant être d'origine animale (porc, cheval, bovins...) ou être produit par culture tissulaire à partir de cellules humaines. De bons résultats ont été obtenus avec du péricarde animal. Toutefois, cette structure tissulaire interne 4 peut-être constituée par une matière synthétique, comme par exemple le polytétrafluoroéthylène expansé.

Sur la figure 1, on a illustré schématiquement l'introduction axiale, selon la flèche F, de la structure interne 4, en position déployée, à l'intérieur de la structure de soutien expansible (stent) 1, elle-même en position expansée.

Lorsque, comme cela est représenté schématiquement sur la figure 2, la structure interne 4 est en place dans la structure de soutien 1, elle couvre au moins en partie la surface interne 7 de ladite structure de soutien 1, c'est-à-dire la surface formée par lesdits fils 2, la surface externe 8 de ladite structure interne 4 restant toutefois accessible depuis l'extérieur à travers les jours formés par les mailles 3 de ladite structure de soutien 1.

La fabrication de l'endoprothèse tissulaire conforme à la présente invention comporte plusieurs étapes :
1. On commence par conformer ladite structure interne 4 à la forme expansée qu'elle doit avoir lorsque la structure de soutien 1 est elle-même expansée (figure 1), par exemple par enroulement sur un mandrin ;
2. Dans le cas où ladite structure tissulaire interne est formée par un tissu biologique, de préférence du péricarde animal, on fixe chimiquement, de façon connue, ce tissu biologique par tout produit approprié tel qu'un aldéhyde. Dans ce dernier cas, on utilise de préférence le glutaraldéhyde, par exemple à la concentration de 0,625 %. Une telle fixation chimique assure au tissu biologique, antigénicité réduite, stabilité chimique, biologique et physique, et notamment résistance aux variations de température et de contraintes mécaniques ;
3A. Ensuite, le tissu biologique de ladite structure interne 4 est lyophilisé. Ce traitement a pour but d'une part de le déshydrater, ce qui est indispensable pour pouvoir le faire adhérer, mais aussi de conserver la structure tridimensionnelle dudit tissu biologique après déshydratation. En effet, lorsqu'un tissu biologique se déshydrate dans des conditions ordinaires, les fibres de collagène qui le constituent viennent au contact les unes des autres et il se crée des liaisons chimiques irréversibles rendant impossible la réhydratation ultérieure du tissu biologique. Pour éviter cet inconvénient, la lyophilisation permet d'immobiliser la structure du tissu biologique par congélation, puis de retirer l'eau à très basse pression par sublimation, donc sans permettre de mobilité et donc de réarrangement des fibres. Les deux paramètres, essentiels au contrôle de la lyophilisation, sont la cinétique et la déshydration :
   ▪ la cinétique doit être très lente - par exemple entre 2°C/heure et 8°C/heure - pour éviter tout risque de surfusion (fusion de l'eau entraînant une réhydratation locale) ;
   ▪ le taux de déshydratation doit être au moins égal à 75 %, de préférence de l'ordre de 78 à 80 %. Un taux de déshydratation trop faible permettrait une certaine réhydratation du tissu biologique et donc une certaine mobilité des fibres qui se traduirait par une perte de souplesse et une mauvaise réhydratation après une phase de stockage (même courte). Une déshydratation trop importante dénaturerait quant à elle le matériau biologique et entraînerait un rétreint important. Les matériaux biologiques sont en effet constitués d'eau liée (10 % de matière sèche et 10 % d'eau liée pour le péricarde) qui entre dans la constitution même du matériau et qu'il ne faut donc pas retirer.
      Pour améliorer encore la préservation de la structure tissulaire pendant la lyophilisation, le tissu biologique est d'abord traité pendant plusieurs jours par un glycol, avantageusement du polyéthylèneglycol, avant d'être lyophilisé. Le polyéthylèneglycol vient en effet créer des liaisons à basse énergie avec les différentes fibres de collagène et donc s'interposer entre ces fibres comme les barreaux d'une échelle. Pendant la lyophilisation, les différentes fibres ne peuvent donc pas venir interagir entre elles. Ces liaisons étant toutefois à basse énergie, le polyéthylène glycol, bien que parfaitement biocompatible (pour certaines masses moléculaires, selon la pharmacopée européenne) est aisément rincé lors de la réhydratation ;
3B. En variante, la lyophilisation peut être remplacée par une déshydratation chimique lente par immersion dudit revêtement interne 4 dans une solution alcoolique de polyéthylèneglycol, de concentration au moins égale à 80 % de polyéthylèneglycol et de 10 % d'alcool, par exemple. Ensuite, la déshydratation est mise en oeuvre à basse pression et à température stable, par exemple 40°C, pendant une durée minimale de 12 heures.
4. Ainsi, grâce à la déshydratation de l'étape 3A ou à celle de l'étape 3B, on obtient une structure tissulaire interne 4 de tissu biologique sec, parfaitement réhydratable, sans altération dudit tissu biologique et quasiment sans rétreint surfacique. Comme illustré schématiquement par la figure 1, cette structure tissulaire interne 4 est alors introduite dans la structure de soutien 1, par exemple à l'aide dudit mandrin (non représenté) sur lequel elle est enroulée. Elle est alors plaquée (par tout moyen connu tel que ballon gonflable, mandrin expansible,... non représenté) contre ladite structure de soutien 1, de façon à former un recouvrement continu, uniforme, sans pli, ni surépaisseur, avec les bords 5A et 5B se touchant rigoureusement pour former une ligne de jonction 6 continue et homogène sans recouvrement, ni espacement. Comme le montre la figure 2, dans cette situation, la surface externe 8 de la structure tissulaire interne 4 en position déployée est alors parfaitement appliquée contre la surface interne 7 de la structure de soutien 1, également en position expansée, cette surface externe 8 restant toutefois accessible depuis l'extérieur à travers les mailles 3.
5. Ensuite, sur l'ensemble structure de soutien 1 - structure tissulaire interne 4 obtenue à l'étape 4 ci-dessus, on forme un revêtement externe 10 emprisonnant la structure de soutien 1, solidarisant cette dernière de la structure tissulaire interne 4, par suite du recouvrement de la surface externe 8 de cette structure tissulaire interne 4 à travers les mailles 3 de ladite structure de soutien 1 et, de plus, obturant la ligne de jonction 6. Ce revêtement externe 10 est formé par déposition d'un agent d'adhésion formé par une dispersion d'une matière synthétique souple et biocompatible dans un solvant dépendant de cette dernière. Cette matière synthétique souple peut être un élastomère de polyuréthane biocompatible implantable et le solvant peut alors être du diméthylacétamide.
   En variante, la matière synthétique souple de ladite dispersion peut être un élastomère de silicone, par exemple du polydiméthylsiloxane biocompatible implantable et le solvant peut alors être du xylène.
   La concentration en poids de matière synthétique dans ladite dispersion est avantageusement comprise entre 10 et 30 %, de préférence entre 20 et 22 %. La viscosité de la dispersion est ajustée entre 500 et 1000 cP en fonction de la nature du tissu biologique de la structure interne 4 et du mode de déposition.
   Le revêtement externe 10 peut être formé, à partir de ladite dispersion par tout moyen connu, par exemple enduction, trempage, coulage ou pulvérisation en fonction de l'état de surface désiré et de la viscosité de la dispersion.
   De préférence, le revêtement externe 10 est réalisé par superposition de plusieurs couches consécutives jusqu'à obtenir l'épaisseur e désirée, qui est par exemple comprise entre 0,1 et 5 mm.
   La surface externe 11 du revêtement externe 10 est continue et, de préférence, présente une microporosité apte à induire une légère fibrose afin de renforcer la liaison mécanique avec la paroi naturelle du patient dans laquelle l'endoprothèse est implantée. Une telle microporosité externe peut être obtenue par pulvérisation d'une dispersion de faible viscosité présentant des inclusions hydrosolubles. La granulométrie de ces inclusions doit être contrôlée afin de maîtriser la taille des pores de la microporosité.
   Éventuellement, la surface externe 11 du revêtement externe 10 peut comporter un ou des relief(s) externe(s) apte(s) à améliorer la tenue mécanique de l'endoprothèse implantée. Toutefois, dans ce cas, il y a lieu de veiller à ce que ce(s) relief(s) n'induise(nt) pas d'érosion cellulaire de la paroi naturelle du patient.
6. Après formation du revêtement externe 10, on procède à l'élimination du solvant de ladite dispersion, par exemple par séchage à chaud, séchage à chaud sous vide et/ou par extraction à chaud dans du sérum physiologique. De préférence, l'élimination du solvant est obtenue par une extraction lente à chaud (par exemple à une température de l'ordre de 40°C) suivie d'une extraction sous vide et complétée par une extraction dans du sérum physiologique.
   Enfin, pendant ou après la phase d'extraction du solvant, le revêtement interne 4 est réhydraté avec du sérum physiologique.

Éventuellement, la face externe 11 du revêtement externe 10 peut être greffée chimiquement avec des peptides, des protéines ou des molécules favorisant l'adhésion cellulaire et de protéines sériques. Cette face externe peut de plus être revêtue d'un agent mouillant ou d'un lubrifiant biocompatible favorisant le glissement de l'endoprothèse lors du chargement du cathéter d'implantation ou du déplacement de mise en place de l'endoprothèse.

Bien que non représenté sur les dessins, au moins un marqueur repérable par imagerie médicale peut être agencé sur l'endoprothèse conforme à la présente invention pour en faciliter l'implantation.

Comme mentionné ci-dessus, l'endoprothèse conforme à la présente invention peut constituer toutes sortes de prothèses, dont celles des valves cardiaques, principalement aortique, mitrale et tricuspide.

Un inconvénient des valves transcatheter connues est une possible fuite para-valvulaire post-implantation, qui dégrade le pronostic médical. Notamment pour les valves aortiques, du fait des calcifications souvent non-homogènes de l'anneau aortique, des espaces peuvent demeurer entre la valve et l'anneau. Pour remédier à cet inconvénient, le revêtement externe 10 d'une valve selon l'invention peut avoir une épaisseur et une souplesse optimisées pour spontanément s'ajuster aux contours du tissu natif ou des calcifications périphériques.

De plus, une épaisseur différente sur la circonférence de la valve selon l'invention, ou dans l'axe longitudinal de celle-ci, peut permettre à la prothèse de valve d'être mieux ajustée à l'anatomie de chaque patient ou de sous-groupes de patients.

L'épaisseur e et la composition du revêtement externe 10 de la prothèse de valve conforme à la présente invention peuvent ne pas être uniformes et varier à différents endroits de ladite prothèse de valve.

## Revendications

1. Endoprothèse tissulaire comportant :
• une structure de soutien expansible creuse (1) à surface ajourée ; et
• une structure interne (4) couvrant au moins en partie la surface interne (7) de ladite structure de soutien (1),
l'endoprothèse comporte un revêtement externe (10) constitué par une matière synthétique souple hémocompatible **caractérisée en ce que** la structure interne est tissulaire et **en ce que** le revêtement externe est ancré mécaniquement à ladite structure tissulaire interne (4), ladite structure de soutien (1) étant emprisonnée au moins partiellement entre ledit revêtement externe (10) et ladite structure tissulaire interne (4), la matière synthétique souple hémocompatible dudit revêtement externe (10) imprégnant au moins partiellement ladite structure tissulaire interne (4).

2. Endoprothèse tissulaire selon la revendication 1,
**caractérisée en ce que** la matière synthétique souple hémocompatible dudit revêtement externe (10) est un élastomère.

3. Endoprothèse tissulaire selon la revendication 2,
**caractérisée en ce que** ledit élastomère est un élastomère de polyuréthane ou de silicone.

4. Endoprothèse tissulaire selon l'une des revendications 1 à 3,
**caractérisée en ce que** la matière synthétique souple hémocompatible dudit revêtement externe (10) est pure ou chargée.

5. Endoprothèse tissulaire selon l'une des revendications 1 à 4,
**caractérisée en ce que** l'épaisseur (e) dudit revêtement interne (10) est au moins de 0,1 mm et au plus de 5 mm.

6. Endoprothèse tissulaire selon l'une des revendications 1 à 5,
**caractérisée en ce que** l'épaisseur (e) dudit revêtement externe (10) est différente à des emplacements différents de ladite endoprothèse.

7. Endoprothèse tissulaire selon l'une des revendications 1 à 6,
**caractérisée en ce que** la structure tissulaire interne est constituée par du péricarde animal.

8. Endoprothèse tissulaire selon l'une des revendications 1 à 6,
**caractérisée en ce que** la structure tissulaire interne est constituée par du polytétrafluoroéthylène expansé.

9. Endoprothèse tissulaire selon l'une des revendications 1 à 8,
**caractérisée en ce qu'**elle forme une prothèse vasculaire.

10. Endoprothèse tissulaire selon l'une des revendications 1 à 8,
**caractérisée en ce qu'**elle forme une prothèse de valve cardiaque.

11. Endoprothèse tissulaire selon l'une des revendications 1 à 10,
**caractérisée en ce que** le revêtement externe (10) possède une surface externe (11), ladite surface externe (11) étant continue et présentant une microporosité apte à induire une légère fibrose.

12. Procédé pour la réalisation d'une endoprothèse tissulaire comportant :
• une structure de soutien expansible creuse (1), à surface ajourée ; et
• une structure tissulaire interne (4) couvrant au moins en partie la surface interne (7) de ladite structure de soutien (1),
**caractérisé en ce que** :
• ladite structure tissulaire interne (4) est introduite dans ladite structure de soutien (1) de façon à couvrir de façon continue, uniforme, sans pli, ni surépaisseur, au moins une partie de la surface interne (7) de ladite structure de soutien (1) ;
• à l'aide d'une dispersion d'une matière synthétique souple hémocompatible dans un solvant, on recouvre la surface de ladite structure de soutien et les parties de la surface externe (8) de ladite structure tissulaire interne (4) accessibles à travers les jours (3) de la surface de ladite structure de soutien (1), pour former un revêtement externe (10) de ladite matière synthétique souple hémocompatible ancré mécaniquement dans ledit revêtement interne (4), la matière synthétique souple hémocompatible dudit revêtement externe (10) imprègne au moins partiellement ladite structure tissulaire interne (4) ; et
• on extrait ledit solvant dudit revêtement externe (10).

13. Procédé selon la revendication 12,
**caractérisé en ce que** ladite matière synthétique du revêtement externe (10) est un élastomère de polyuréthane ou de silicone.

14. Procédé selon l'une des revendications 12 ou 13,
**caractérisé en ce que** la concentration en poids de ladite matière synthétique dans la dispersion est comprise entre 10 et 30 %.

15. Procédé selon la revendication 14,
**caractérisé en ce que** la concentration en poids de ladite matière synthétique dans la dispersion est comprise entre 20 et 22 %.

16. Procédé selon l'une des revendications 12 à 15,
**caractérisé en ce que** la viscosité de ladite dispersion est comprise entre 500 et 1000 cP.

17. Procédé selon l'une des revendications 12 à 16, dans lequel ladite structure tissulaire interne (4) est formée par un tissu biologique fixé chimiquement,
**caractérisé en ce que** ladite structure tissulaire interne (4) :
• est soumise à une déshydratation partielle après fixation chimique et avant introduction dans ladite structure de soutien (1) ; puis
• est réhydratée pendant ou après l'extraction du solvant dudit revêtement externe (10).

## Patentansprüche

1. Gewebe-Endoprothese, umfassend:
• eine hohle expansible Stützstruktur (1) mit durchbrochener Fläche; und
• eine Innenstruktur (4), die die Innenfläche (7) der Stützstruktur (1) mindestens zum Teil bedeckt,
die Endoprothese umfasst eine Außenbeschichtung (10), die aus einem hämokompatiblen weichen synthetischen Material zusammengesetzt ist, **dadurch gekennzeichnet, dass** die Innenstruktur Gewebe ist und dadurch, dass die Außenbeschichtung mechanisch an der Gewebe-Innenstruktur (4) verankert ist, wobei die Stützstruktur (1) mindestens teilweise zwischen der Außenbeschichtung (10) und der Gewebe-Innenstruktur (4) eingesperrt ist, wobei das hämokompatible weiche synthetische Material der Außenbeschichtung (10) die Gewebe-Innenstruktur (4) mindestens teilweise imprägniert.

2. Gewebe-Endoprothese nach Anspruch 1,
**dadurch gekennzeichnet, dass** das hämokompatible weiche synthetische Material der Außenbeschichtung (10) ein Elastomer ist.

3. Gewebe-Endoprothese nach Anspruch 2,
**dadurch gekennzeichnet, dass** das Elastomer ein Polyurethan- oder Silikonelastomer ist.

4. Gewebe-Endoprothese nach einem der Ansprüche 1 bis 3,
**dadurch gekennzeichnet, dass** das hämokompatible weiche synthetische Material der Außenbeschichtung (10) pur oder beladen ist.

5. Gewebe-Endoprothese nach einem der Ansprüche 1 bis 4,
**dadurch gekennzeichnet, dass** die Dicke (e) der Innenbeschichtung (10) mindestens 0,1 mm und höchstens 5 mm beträgt.

6. Gewebe-Endoprothese nach einem der Ansprüche 1 bis 5,
**dadurch gekennzeichnet, dass** sich die Dicke (e) der Außenbeschichtung (10) an unterschiedlichen Stellen der Endoprothese unterscheidet.

7. Gewebe-Endoprothese nach einem der Ansprüche 1 bis 6,
**dadurch gekennzeichnet, dass** die Gewebe-Innenstruktur aus tierischem Perikard zusammengesetzt ist.

8. Gewebe-Endoprothese nach einem der Ansprüche 1 bis 6,
**dadurch gekennzeichnet, dass** die Gewebe-Innenstruktur aus expandiertem Polytetrafluorethylen zusammengesetzt ist.

9. Gewebe-Endoprothese nach einem der Ansprüche 1 bis 8,
**dadurch gekennzeichnet, dass** sie eine Gefäßprothese bildet.

10. Gewebe-Endoprothese nach einem der Ansprüche 1 bis 8,
**dadurch gekennzeichnet, dass** sie eine Herzklappenprothese bildet.

11. Gewebe-Endoprothese nach einem der Ansprüche 1 bis 10,
**dadurch gekennzeichnet, dass** die Außenbeschichtung (10) eine Außenfläche (11) besitzt, wobei die Außenfläche (11) durchgehend ist und eine Mikroporosität aufweist, die eine leichte Fibrose induzieren kann.

12. Verfahren für die Herstellung einer Gewebe-Endoprothese, umfassend:
• eine hohle expansible Stützstruktur (1) mit durchbrochener Fläche; und
• eine Gewebe-Innenstruktur (4), die die Innenfläche (7) der Stützstruktur (1) mindestens zum Teil bedeckt,
**dadurch gekennzeichnet, dass**:
• die Gewebe-Innenstruktur (4) so in die Stützstruktur (1) eingeführt wird, dass sie mindestens einen Teil der Innenfläche (7) der Stützstruktur (1) in durchgehender, einheitlicher Weise ohne Falten und Überdicken bedeckt;
• die Fläche der Stützstruktur und die Teile der Außenfläche (8) der Gewebe-Innenstruktur (4), die durch die Durchbrüche (3) der Fläche der Stützstruktur (1) hindurch zugänglich sind, mithilfe einer Dispersion eines hämokompatiblen weichen synthetischen Materials in einem Lösungsmittel überzogen wird, um eine Außenbeschichtung (10) des hämokompatiblen weichen synthetischen Materials zu bilden, die mechanisch in der Innenbeschichtung (4) verankert ist, wobei das hämokompatible weiche synthetische Material der Außenbeschichtung (10) die Gewebe-Innenstruktur (4) mindestens teilweise imprägniert; und
• das Lösungsmittel aus der Außenbeschichtung (10) entzogen wird.

13. Verfahren nach Anspruch 12,
**dadurch gekennzeichnet, dass** das synthetische Material der Außenbeschichtung (10) ein Polyurethan- oder Silikonelastomer ist.

14. Verfahren nach einem der Ansprüche 12 oder 13,
**dadurch gekennzeichnet, dass** die Gewichtskonzentration des synthetischen Materials in der Dispersion zwischen 10 und 30 % beträgt.

15. Verfahren nach Anspruch 14,
**dadurch gekennzeichnet, dass** die Gewichtskonzentration des synthetischen Materials in der Dispersion zwischen 20 und 22 % beträgt.

16. Verfahren nach einem der Ansprüche 12 bis 15,
**dadurch gekennzeichnet, dass** die Viskosität der Dispersion zwischen 500 und 1000 cP beträgt.

17. Verfahren nach einem der Ansprüche 12 bis 16, wobei die Gewebe-Innenstruktur (4) von einem chemisch fixierten biologischen Gewebe gebildet wird,
**dadurch gekennzeichnet, dass** die Gewebe-Innenstruktur (4):
• nach chemischer Fixierung und vor Einführen in die Stützstruktur (1) einer teilweisen Dehydratisierung ausgesetzt wird; und anschließend
• während oder nach dem Entzug des Lösungsmittels aus der Außenbeschichtung (10) rehydratisiert wird.

## Claims

1. Tissue endoprosthesis comprising:
• an expandable hollow support structure (1) having a surface with openings; and
• an inner structure (4) at least partly covering the inner surface (7) of said support structure (1),
the endoprosthesis comprises an outer covering (10) which is composed of a flexible haemocompatible synthetic material
**characterised in that** the inner structure (4) is an inner tissue structure and **in that** the outer covering is anchored mechanically to said inner tissue structure (4), said support structure (1) being held at least partially between said outer covering (10) and said inner tissue structure (4), the flexible haemocompatible synthetic material of said outer covering (10) impregnating said inner tissue structure (4) at least partially.

2. Tissue endoprosthesis according to claim 1,
**characterised in that** the flexible haemocompatible synthetic material of said outer covering (10) is an elastomer.

3. Tissue endoprosthesis according to claim 2,
**characterised in that** said elastomer is a polyurethane elastomer or a silicone elastomer.

4. Tissue endoprosthesis according to any of claims 1 to 3,
**characterised in that** the flexible haemocompatible synthetic material of said outer covering (10) is pure or loaded.

5. Tissue endoprosthesis according to any of claims 1 to 4,
**characterised in that** the thickness (e) of said outer covering (10) is at least 0.1 mm and at most 5 mm.

6. Tissue endoprosthesis according to any of claims 1 to 5,
**characterised in that** the thickness (e) of said outer covering (10) is different at different locations of said endoprosthesis.

7. Tissue endoprosthesis according to any of claims 1 to 6,
**characterised in that** the inner tissue structure is composed of animal pericardium.

8. Tissue endoprosthesis according to any of claims 1 to 6,
**characterised in that** the inner tissue structure is composed of expanded polytetrafluoroethylene.

9. Tissue endoprosthesis according to any of claims 1 to 8,
**characterised in that** it forms a vascular prosthesis.

10. Tissue endoprosthesis according to any of claims 1 to 8,
**characterised in that** it forms a cardiac valve prosthesis.

11. Tissue endoprosthesis according to any of claims 1 to 10,
**characterised in that** the outer covering (10) has an outer surface (11), said outer surface (11) being continuous and having a microporosity capable of inducing slight fibrosis.

12. Method for the production of a tissue endoprosthesis comprising:
• an expandable hollow support structure (1) having a surface with openings; and
• an inner tissue structure (4) at least partly covering the inner surface (7) of said support structure (1),
**characterised in that**:
• said inner tissue structure (4) is introduced into said support structure (1) so as to cover, in a continuous, uniform manner, without folding or excessive thickness, at least a portion of the inner surface (7) of said support structure (1);
• by means of a dispersion of a flexible haemocompatible synthetic material in a solvent, the surface of said support structure and the portions of the outer surface (8) of said inner tissue structure (4) that are accessible through the openings (3) in the surface of said support structure (1) are covered in order to form an outer covering (10) of said flexible haemocompatible synthetic material which is anchored mechanically in said inner covering (4), the flexible haemocompatible synthetic material of said outer covering (10) impregnates said inner tissue structure (4) at least partially; and
• said solvent is extracted from said outer covering (10).

13. Method according to claim 12,
**characterised in that** said synthetic material of the outer covering (10) is a polyurethane elastomer or a silicone elastomer.

14. Method according to either claim 12 or claim 13,
**characterised in that** the concentration by weight of said synthetic material in the dispersion is between 10 and 30 %.

15. Method according to claim 14,
**characterised in that** the concentration by weight of said synthetic material in the dispersion is between 20 and 22 %.

16. Method according to any of claims 12 to 15,
**characterised in that** the viscosity of said dispersion is between 500 and 1000 cP.

17. Method according to any of claims 12 to 16, wherein said inner tissue structure (4) is formed by a chemically fixed biological tissue,
**characterised in that** said inner tissue structure (4):
• is subjected to partial dehydration after chemical fixing and before introduction into said support structure (1); and then
• is rehydrated during or after the extraction of the solvent from said outer covering (10).
